# EUROPEAN PATENT SPECIFICATION

(11) **EP 2 885 023 B1**
(45) Date of publication and mention of the grant of the patent: **04.07.2018**
(21) Application number: 13829796.5
(22) Date of filing: 13.08.2013
(51) Int. Cl.: A61M 5/178, A61M 5/48, A61M 5/31, A61M 5/145

(54) **SYRINGE**
SPRITZE
SERINGUE

(30) Priority: 15.08.2012 US 201261683625 P
(43) Date of publication of application: 24.06.2015
(73) Proprietor: Coeur, Inc., Lebanon TN 37087 (US)
(72) Inventor: CUDE, Michael, J., College Grove, Tennessee 37046 (US)
(74) Representative: Trinks, Ole
(86) International application number: PCT/US2013/054676
(87) International publication number: WO 2014/028452

(56) References cited:
- US-A- 3 967 759
- US-A- 3 987 940
- US-A- 4 702 737
- US-A1- 2004 122 369
- US-A1- 2010 280 369
- US-A1- 2012 053 529

## Description

### CROSS-REFERENCE TO RELATED APPLICATION

This application claims priority to and the benefit of United States Provisional Application No. 61/683,625, filed August 15, 2012.

### BACKGROUND

This invention relates in general to medical injection systems.

One type of medical injection system includes a syringe and a power-driven injector. For example, for intravenous delivery the syringe may be filled with fluid to delivery and the syringe may be connected to a patient by appropriate tubing and a needle. Typically, in such an arrangement, the injector will include a plunger which engages a plunger jacket inside the syringe to push fluid from the syringe to a patient.

Such syringes are typically made of plastic. Due to the high pressures generated during the delivery process such syringes may include an outer pressure jacket. Alternatively, a syringe with a relatively thicker outer wall may be used and then not include an outer pressure jacket. US2004/0122369 A1 discloses a syringe with a pressure jacket and a thinner wall in a proximal section of its barrel in order to overcome disadvantages related to swelling due to creep over time and during sterilization.

### SUMMARY

This invention relates more specifically to syringes for medical injection systems.

In one embodiment according to the invention a syringe for a power injector includes a barrel having a distal end and a proximal end. The barrel defines an interior volume therebetween the distal and proximal ends. A circumferential flange extends about the proximal end. A delivery tip extends from the distal end. The barrel includes a thick wall section adjacent the distal end and a thin wall section adjacent the proximate end.

The syringe defines a common inner surface with the same inner diameter for engagement of a plunger jacket shared by the thick wall section and the thin wall section. In addition, the syringe is a jacketless syringe.

The syringe may be formed from thermo-plastic, such as polypropylene.

The thick wall section may have a greater pressure capability than the thin wall section. The thick wall section may have a pressure capability of at least 460 psi. The thin wall section may have a pressure capability of at least 375 psi.

Various aspects will become apparent to those skilled in the art from the following detailed description and the accompanying drawings.

### BRIEF DESCRIPTION OF THE DRAWINGS

Fig. 1 is a perspective view of a injection system as shown in exploded view.
Fig. 2 is an enlarged perspective view of the syringe of Fig. 1.
Fig. 3 is a cross-sectional view of the syringe of Fig. 2.

### DETAILED DESCRIPTION

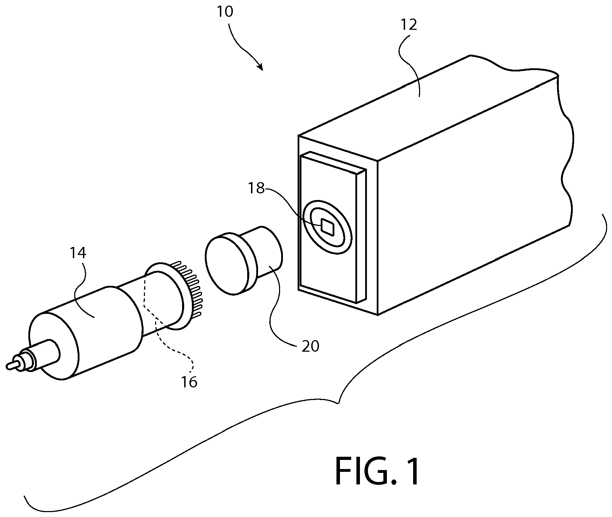

Referring now to the drawings, there is illustrated in Fig. 1 an injection system 10. The system 10 includes a power driven injector 12 and a syringe 14. A plunger jacket 16 is optionally disposed within the syringe 14. The injector 12 includes a ram 18 that drives a plunger 20 that engages the plunger jacket 16 and thus may push fluid from the syringe 14.

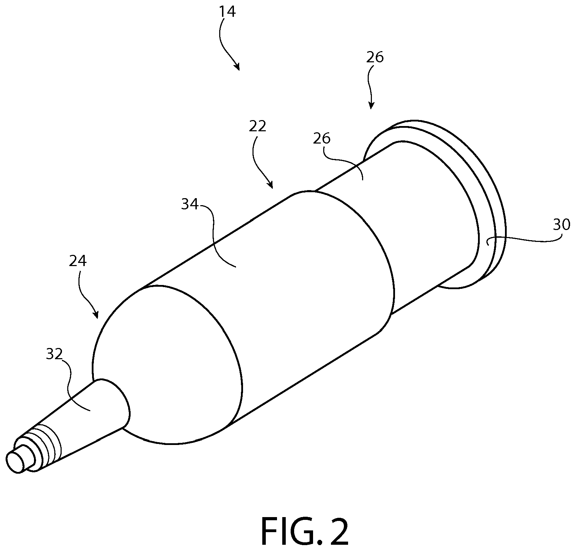

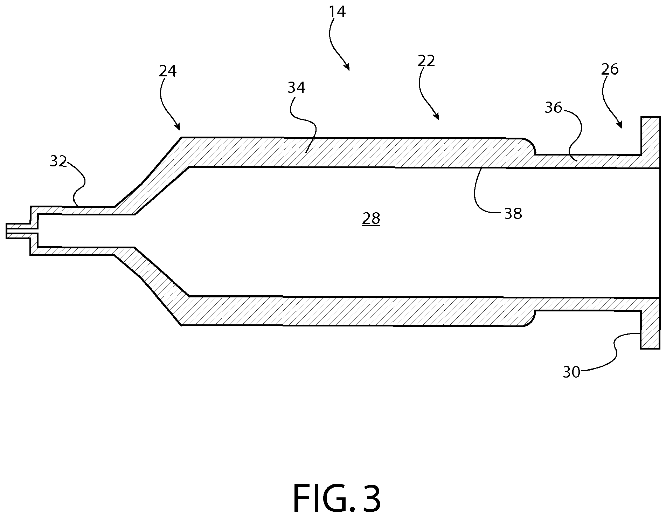

As best shown in Figs 2 and 3, the syringe 14 includes a barrel 22. The barrel 22 has a distal end 24 and a proximal end 26. The barrel 22 defines an interior volume 28 therebetween the distal end 24 and the proximal end 26. A circumferential flange 30 extends about the proximal end 26. A delivery tip 32 extends from the distal end 24.

The barrel 22 includes a thick wall section 34 adjacent the distal end 24. The barrel 22 includes a thin wall section 36 adjacent the proximate end 26. The thick wall section 34 and the thin wall section 36 are generally thick and thin respectively relative to one another. The thick wall section 34 and the thin wall section 36 share a common inner surface 38 for engagement of the plunger jacket 16.

In one embodiment the syringe 14 is formed from an olefin or thermo-plastic, such as Polypropylene.

The thick wall section 34 has a greater pressure capability than the thin wall section 36. For example the thick wall section 34 may be rated to 460 psi while the thin wall section 36 is rated for 375 psi. Thus in at least one embodiment the thick wall section may have a pressure capability of at least 460 psi, and in at least one embodiment the thin wall section may have a pressure capability of at least 375 psi.

It has been discovered that the pressure of a fluid in the interior volume 28 increases as the plunger 20 and plunger jacket 16 move along the axial length of the barrel 22 from the proximal end 26 toward the distal end 24. In operation the syringe 14 with a syringe jacket, i.e. a jacketless syringe, may withstand greater pressure in use than an otherwise thinner unjacketed syringe, while having a relatively thinner wall section adjacent the proximate end.

In one operation, the syringe 14 is filled with a fluid for an injection operation. The syringe 14 with plunger jacket 16, the plunger 20, and without a syringe jacket, is loaded on the power driven injector 12 with the ram 18 engaging the plunger 20 and the plunger jacket 16 to push fluid from the syringe 14 with the pressure in the syringe increasing as the ram 18 moves along the syringe 14.

While principles and modes of operation have been explained and illustrated with regard to particular embodiments, it must be understood, however, that this may be practiced otherwise than as specifically explained and illustrated without departing from its scope.

## Claims

1. A syringe (14) for a medical power injector (12) comprising:
a barrel (22) having a distal end (24) and a proximal end (26) and defining an interior volume (28) therebetween,
a delivery tip (32) extending from the distal end (24),
wherein the barrel (22) includes a thick wall section (34) adjacent the distal end (24) and a thin wall section (36) adjacent the proximal end (26),
**characterized in that**
the syringe (14) further comprises a circumferential flange (30) extending about the proximal end (26), and that the syringe (14) is a jacketless syringe, wherein the thick wall section (34) and the thin wall section (36) have the same inner diameter.

2. A medical system (10) for injecting a fluid comprising:
a syringe (14) as defined in claim 1; and
a power injector (12) with a ram (18) for pushing fluid from the syringe (14).

3. The system of claim 2 further comprising:
a plunger (20) and a plunger jacket (16) engaging the ram (18) and disposed within the syringe (14).

4. The system of claim 3 wherein the barrel (22) defines a common inner surface (38) for engagement of the plunger jacket (16) shared by the thick wall section (34) and the thin wall section (36).

5. The system of one of claims 2 to 4, wherein the syringe (14) is formed from thermo-plastic.

6. The system of claim 5 wherein the thermo-plastic is polypropylene.

7. The system of one of claims 2 to 6 wherein the thick wall section (34) has a greater pressure capability than the thin wall section.

8. The system of one of claims 2 to 7 wherein the thick wall section (34) has a pressure capability of at least 3,17 MPa (460 psi).

9. The system of one of claims 2 to 8 wherein the thin wall section (36) has a pressure capability of at least 2,59 MPa (375 psi).

10. A method for operating the medical injector system (10) of claims 2 to 9, comprising:
loading the syringe (14) on the power injector (12).

11. The method of claim 10 further comprising, prior to loading the syringe (14) on the power injector (12), filling the syringe (14) with a fluid for injection.

12. The method of claim 10 wherein the loading includes loading the syringe (14) on the power injector (12) with a plunger (20) and a plunger jacket (16) engaging the ram (18).

## Patentansprüche

1. Spritze (14) für einen medizinischen Power-Injektor (12), umfassend:
einen Zylinder (22), der ein distales Ende (24) und ein proximales Ende (26) aufweist und ein Innenvolumen (28) dazwischen definiert,
eine Abgabespitze (32), die sich vom distalen Ende (24) erstreckt,
wobei der Zylinder (22) einen dicken Wandabschnitt (34) neben dem distalen Ende (24) und einen dünnen Wandabschnitt (36) neben dem proximalen Ende (26) aufweist,
**dadurch gekennzeichnet, dass**
die Spritze (14) ferner einen Umfangsflansch (30) umfasst, der sich um das proximale Ende (26) erstreckt, und dass die Spritze (14) eine mantellose Spritze ist, wobei der dicke Wandabschnitt (34) und der dünne Wandabschnitt (36) denselben Innendurchmesser aufweisen.

2. Medizinisches System (10) zum Injizieren einer Flüssigkeit, umfassend:
eine Spritze (14) nach Anspruch 1; und
einen Power-Injektor (12) mit einem Stößel (18) zum Schieben der Flüssigkeit aus der Spritze (14).

3. System nach Anspruch 2, ferner umfassend:
einen Kolben (20) und einen Kolbenmantel (16), der den Stößel (18) in Eingriff nimmt und in der Spritze (14) angeordnet ist.

4. System nach Anspruch 3, wobei der Zylinder (22) eine gemeinsame Innenfläche (38) für einen Eingriff mit dem Kolbenmantel (16) definiert, die dem dicken Wandabschnitt (34) und dem dünnen Wandabschnitt (36) gemeinsam ist.

5. System nach einem der Ansprüche 2 bis 4, wobei die Spritze (14) aus einem thermoplastischen Material besteht.

6. System nach Anspruch 5, wobei das thermoplastische Material Polypropylen ist.

7. System nach einem der Ansprüche 2 bis 6, wobei der dicke Wandabschnitt (34) eine größere Drucktragfähigkeit als der dünne Wandabschnitt aufweist.

8. System nach einem der Ansprüche 2 bis 7, wobei der dicke Wandabschnitt (34) eine Drucktragfähigkeit von mindestens 3,17 MPa (460 psi) aufweist.

9. System nach einem der Ansprüche 2 bis 8, wobei der dünne Wandabschnitt (36) eine Drucktragfähigkeit von mindestens 2,59 MPa (375 psi) aufweist.

10. Verfahren zur Bedienung des medizinischen Injektorsystems (10) nach den Ansprüchen 2 bis 9, umfassend:
Laden der Spritze (14) auf den Power-Injektor (12).

11. Verfahren nach Anspruch 10, ferner umfassend, vor dem Laden der Spritze (14) auf den Power-Injektor (12), das Füllen der Spritze (14) mit einer Flüssigkeit zur Injektion.

12. Verfahren nach Anspruch 10, wobei das Laden das Laden der Spritze (14) auf den Power-Injektor (12) umfasst, wobei ein Kolben (20) und ein Kolbenmantel (16) den Stößel (18) in Eingriff nehmen.

## Revendications

1. Seringue (14) destinée à un injecteur électromédical (12) comprenant :
un corps (22) ayant une extrémité distale (24) et une extrémité proximale (26) et délimitant un volume intérieur (28) entre celles-ci,
une pointe d'administration (32) s'étendant depuis l'extrémité distale (24),
dans laquelle le corps (22) comprend une section paroi épaisse (34) adjacente à l'extrémité distale (24) et une section paroi mince (36) adjacente à l'extrémité proximale (26),
**caractérisée en ce que**
la seringue (14) comprend en outre une collerette circonférentielle (30) s'étendant autour de l'extrémité proximale (26), et **en ce que** la seringue (14) est une seringue sans enveloppe, la section paroi épaisse (34) et la section paroi mince (36) ayant le même diamètre interne.

2. Système médical (10) permettant d'injecter un fluide comprenant :
une seringue (14) telle que définie dans la revendication 1 ; et
un injecteur électrique (12) doté d'un plongeur (18) pour pousser le fluide présent dans la seringue (14).

3. Système selon la revendication 2 comprenant en outre :
un piston (20) et une enveloppe (16) de piston entrant en prise avec le plongeur (18) et disposés à l'intérieur de la seringue (14).

4. Système selon la revendication 3 dans lequel le corps (22) délimite une surface interne commune (38) pour l'engrènement de l'enveloppe (16) de piston partagée par la section paroi épaisse (34) et la section paroi mince (36).

5. Système selon l'une des revendications 2 à 4, dans lequel la seringue (14) est formée à partir de thermoplastique.

6. Système selon la revendication 5 dans lequel le thermoplastique est du polypropylène.

7. Système selon l'une des revendications 2 à 6 dans lequel la section paroi épaisse (34) a une capacité de pression supérieure à celle de la section paroi mince.

8. Système selon l'une des revendications 2 à 7 dans lequel la section paroi épaisse (34) a une capacité de pression d'au moins 3,17 MPa (460 psi).

9. Système selon l'une des revendications 2 à 8 dans lequel la section paroi mince (36) a une capacité de pression d'au moins 2,59 MPa (375 psi).

10. Procédé permettant d'utiliser le système médical d'injection (10) selon les revendications 2 à 9, comprenant :
le chargement de la seringue (14) sur l'injecteur électrique (12).

11. Procédé selon la revendication 10 comprenant en outre, avant le chargement de la seringue (14) sur l'injecteur électrique (12), le remplissage de la seringue (14) avec un fluide pour injection.

12. Procédé selon la revendication 10 dans lequel le chargement consiste à charger la seringue (14) sur l'injecteur électrique (12) avec un piston (20) et une enveloppe (16) de piston en prise avec le plongeur (18).
